(19) Europäisches Patentamt
European Patent Office
Office européen des brevets

(11) Veröffentlichungsnummer: **0 002 184**
**A1**

(12) # EUROPÄISCHE PATENTANMELDUNG

(21) Anmeldenummer: **78101129.1**

(22) Anmeldetag: **13.10.78**

(51) Int. Cl.[2]: **A 61 K 7/16**

(30) Priorität: **26.11.77 DE 2752852**

(43) Veröffentlichungstag der Anmeldung: **13.06.79 Patentblatt 79/12**

(84) Benannte Vertragsstaaten: **BE CH DE FR GB LU NL SE**

(71) Anmelder: **Giulini Chemie GmbH**
**Giulinistrasse 2**
**D-6700 Ludwigshafen/Rhein(DE)**

(72) Erfinder: **Vogt, Curt**
**Rubensstrasse 31**
**D-6700 Ludwigshafen/Rhein(DE)**

(72) Erfinder: **Mattes, Ludwig, Ing.-Grad.**
**Rabengasse 6**
**D-6800 Mannheim 31(DE)**

(74) Vertreter: **Benatzky, Erika, Dr.**
**Giulinistrasse 2**
**D-6700 Ludwigshafen/Rh.(DE)**

(54) **Wirkungsverstärker für Zahnreinigungsmittel.**

(57) Verwendung von Polyphosphat-Feingranulat der allgemeinen Formel

$Na_{n+2}P_nO_{3n+1}$

in der $n \geq 3$ ist, zur schnelleren Beseitigung von Zahnstein und/oder Zahnverfärbungen bei Rauchern, gegebenenfalls als Wirkungsverstärker in Kombination mit Zahnreinigungsmitteln.

EP 0 002 184 A1

Croydon Printing Company Ltd.

BEZEICHNUNG GEÄNDERT Pat 327 EU
siehe Titelseite

Zahnbehandlungsmittel (Verstärker für Zahnpflegemittel)

Vorstehende Erfindung betrifft ein Mittel auf Basis feingranulierter Polyphosphate zur Wirkungsverstärkung von Zahnreinigungsmitteln, insbesondere Zahnpasten, wie auch zur alleinigen Anwendung gegen verstärkte Zahnsteinbildungen und rauchbedingte Zahnverfärbungen.

Zahnstein verhindernde bzw. lösende Zahnbehandlungsmittel wurden bereits mehrfach in der Literatur beschrieben. So ist schon vorgeschlagen worden, neben den in Zahnpasten üblicherweise enthaltenden Schleifmitteln wie beispielsweise Calciumcarbonat, Dicalciumphosphat, Tricalciumphosphat, Calciumpyrophosphat, Calciumsulfat, Bentonit, unlösliches Natriummetaphosphat, Kieselsäure, Aluminiumhydroxid oder -oxid, Tensiden, Bindemitteln, Fluorverbindungen, Geschmacksstoffen u. a. noch solche Stoffe zuzugeben, die in der Lage sind, die Calciumionen des Zahnsteines oder des sich bildenden Zahnsteines zu binden. Derartige Komplexbildner, z. B. Polyphosphate, Phosphonsäuren und/oder deren Salze, Polycarbonsäuren u. a. sollen gemäß den vorerwähnten Vorschlägen in einer Menge von 2 bis 5 % in die Zahnpasten eingearbeitet werden.

Langjährige Beobachtungen und Untersuchungen haben aber gezeigt, daß die Bildung von Zahnstein sich sehr unterschiedlich

lleicht und die Zahnverfärbungen bei Rauchern oftmals hwer beeinflußbar sind. Insbesondere auf der Rückseite von rende- und Backenzähnen und in den Zahnzwischenräumen sich leicht lästiger Zahnstein an, der selbst bei zwei- l täglicher Zahnpflege nicht entfernt werden kann. Ernäh- ngsgewohnheiten, Hartwasserverhältnisse, individuell ver- schiedenartige Disposition mögen zusätzlich zu dem Steinan- tz beitragen. Der Reinigungsvorgang mit Bürste und Reini- ngsmittel vollzieht sich schließlich in der Regel in nur nr kurzer Zeit, so daß die Einwirkungsmöglichkeit durch das inigungsmittel sowie mechanisch durch die Bürste nicht ge- gt.

e handelsüblichen Zahnreinigungs- und Pflegemittel sind auf rchschnittliche Bedürfnisse eingestellt und können den Ver- ltnissen besonders starker Verfärbungen und Zahnsteinbil- ngen nicht Rechnung tragen. Es stellte sich somit die Auf- gabe, Mittel zu finden, mit deren Hilfe eine zeitweilige Ver- rkung der Zahnreinigung ohne Schädigung der Zahnsubstanz einer technisch bequemen Form möglich ist.

Erfindungsgemäß wird die Aufgabe mit einem Zahnbehandlungsmittel gelöst, das aus einem Polyphosphat-Feingranulat der allgemeinen Formel $Na_{n+2}P_nO_{3n+1}$, in der $n \geqq 3$ ist, besteht. Der maximale Wert von n beträgt 30, bevorzugt liegt er jedoch zwischen 10 und 25. Mit Vorteil werden hierbei solche Polyphosphatgranulate verwendet, wie sie in der deutschen Patentschrift 1 097 963 beschrieben werden. Bei diesen Phosphaten handelt es sich um freifließende, nicht hygroskopische, kondensierte glasige Phosphate, die unter Verwendung von flüssigen und festen Granulierhilfsmitteln in der Weise hergestellt werden, daß pulverförmige, kondensierte Phosphate zunächst mit einem flüssigen Granulierhilfsmittel, z. B. Wasser und/oder einer flüssigen bzw. in Wasser gelösten oberflächenaktiven Substanz granuliert und anschließend mit

einem festen Granulierhilfsmittel versetzt werden, wobei als festes Granulierhilfsmittel ebenfalls ein Polyphosphat eingesetzt werden kann.

Das Feingranulat erfüllt die notwendige Voraussetzung, daß es selbst bei üblicher Aufbewahrung in Kleinverpackung auch unter den Feuchtigkeitsverhältnissen von Badezimmern gut rieselfähig bleibt, also nicht hygroskopisch ist. Prinzipiell könnte die Zahnbehandlung auch mit einer wäßrigen, kurz vor der Behandlung hergestellten Lösung der Feingranulate vorgenommen werden, jedoch ist hierbei der Erfolg geringer.

Die praktische Anwendung des neuen Zahnbehandlungsmittels kann mit Vorteil so geschehen, daß eine geringe Menge des Polyphosphat-Feingranulates, z. B. 0,1 bis 1 g, auf eine Zahnbürste aufgetragen und damit die Zähne gebürstet werden. Hierbei ergibt sich im ersten Moment eine gewisse Scheuerwirkung, wobei sich dann das Feingranulat auflöst. Das gelöste Polyphosphat greift den Zahnstein chemisch an. Durch die kombinierte mechanisch-chemische Behandlung erfolgt überraschenderweise ein Aufbrechen des vorhandenen Zahnsteines. Um den beschriebenen Effekt zu erreichen, kann die Behandlung der Zähne auch in Verbindung mit einer handelsüblichen Zahnpaste vorgenommen werden. Auch ist es möglich, die Polyphosphat-Feingranulate mit weiteren scheuernd wirkenden Produkten zu kombinieren. Gleichzeitig können auch andere, in der Literatur beschriebene Hilfsstoffe für die Herstellung von Zahn- und Mundpflegemitteln verwendet werden. Hierzu gehören z. B. Tenside, Fluorverbindungen wie Dinatrium-Monofluorphosphat, Kresotinsäure, Benzoesäure, Salizylsäure bzw. die entsprechenden Ester. Weiterhin können Geschmacks- und Geruchsstoffe sowie heilwirksame Pflanzenauszüge wie z. B. Azulen, Kamille, Pfefferminzöl u. a. zugegeben werden.

Beispiel 1

Auf eine Zahnbürste wird ein Streifen Zahnpaste von 15 mm Länge aufgetragen. Anschließend werden 0,15 g Polyphosphat-Feingranulat aufgestreut. Anschließend werden die Zähne wie üblich geputzt. Während bei alleiniger Anwendung von Zahnpaste innerhalb von 2 Monaten noch deutlich Zahnsteinbeläge, besonders zwischen den Zähnen und an der Zahnrückseite zu erkennen waren, sind bei dem erfindungsgemäßen Verfahren die Zähne frei von Zahnstein.

Beispiel 2

Auf die angefeuchtete Zahnbürste direkt 0,10 bis 0,20 g Polyphosphat-Feingranulat aufgebracht. Hiermit werden an einigen aufeinander folgenden Tagen die Zähne zusätzlich gereinigt, um dann wieder auf alleinige Zahnpasten-Verwendung überzugehen.

Pat 327 EU

## Patentansprüche

1) Mittel zur Wirkungsverstärkung von Zahnreinigungsmitteln zur schnelleren Beseitigung von Zahnstein und/oder Zahnverfärbungen bei Rauchern dadurch gekennzeichnet, daß es aus einem Polyphosphat-Feingranulat der allgemeinen Formel

$$Na_{n+2}P_nO_{3n+1}$$

in der $n \geqq 3$ ist, besteht.

2) Mittel nach Anspruch 1, dadurch gekennzeichnet, daß der maximale Wert von n 30, bevorzugt jedoch 10 bis 25 ist.

3) Mittel gemäß den Ansprüchen 1 bis 2, dadurch gekennzeichnet, daß es zusätzlich desinfizierende, geschmacksverbessernde und im Granulierverfahren eingearbeitete Zusätze enthält.

4) Mittel gemäß den Ansprüchen 1 bis 3, dadurch gekennzeichnet, daß es zur besseren Unterscheidung zu Zahnpasten und Zahnreinigungspulvern eingefärbt ist.

0002184

Europäisches Patentamt

# EUROPÄISCHER RECHERCHENBERICHT

Nummer der Anmeldung
EP 78 10 1129

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 2 191 199 (HALL)<br>* Seite 1, linke Spalte, Zeilen 1-56; Seite 1, rechte Spalte, Zeile 34 - Seite 2, linke Spalte Zeile 35; Seite 2, rechte Spalte Zeilen 10-37; Beispiele; Ansprüche *<br>-- | 1-4 |
| X | DE - A - 2 602 981 (CARTER-WALLACE)<br>* Seite 2, Zeilen 1-7; Seite 4, Zeilen 1-21; Seite 7, Zeilen 1-4; Ansprüche *<br>-- | 1-4 |
| | FR - A - 1 483 486 (KNAPSACK)<br>* Seite 1, linke Spalte, Zeile 1 bis rechte Spalte, Zeile 33; Zusammenfassung *<br>-- | 1-4 |
| A | FR - A - 2 130 275 (GENERAL FOODS)<br>* Ansprüche *<br>-- | 1 |
| A | FR - A - 2 015 038 (BLENDAX)<br>* Seite 1, Zeilen 1-30 *<br>-- | 1 |
| A | FR - A - 2 070 065 (PRODENTA)<br>* Ansprüche *<br>---- | 1 |

KLASSIFIKATION DER ANMELDUNG (Int.Cl.²)
A 61 K 7/16

RECHERCHIERTE SACHGEBIETE (Int. Cl.²)
A 61 K 7/16

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 02-03-1979 | JONAS |